# FASCICULE DE BREVET EUROPEEN

(11) **EP 2 737 947 B1**
(45) Date de publication et mention de la délivrance du brevet: **21.08.2019**
(21) Numéro de dépôt: 13194364.9
(22) Date de dépôt: 26.11.2013
(51) Int. Cl.: B01J 37/02, B01J 37/34, B01J 23/755, B01J 23/40, C10G 45/40, B01J 23/42, B01J 23/44, C10G 45/34

(54) **Utilisation de catalyseurs métalliques supportés pour l'hydrogénation d'hydrocarbures insaturés**
Vervendung von Metallkatalysatoren zur Hydrierung von ungesättigten Kohlenwasserstoffen
Use of supported metal catalysts for the hydrogenation of unsaturated hydrocarbons

(30) Priorité: 30.11.2012 FR 1261449; 07.12.2012 US 201261734709 P
(43) Date de publication de la demande: 04.06.2014
(73) Titulaire: Eurecat S.A., 07800 La Voulte-sur-Rhône (FR)
(72) Inventeur: DUFRESNE, Pierre, 26000 VALENCE (FR); KIRUMAKKI, Sharath, FRIENDSWOOD, TEXAS, 77546 (US)
(74) Mandataire: Zapalowicz, Francis

(56) Documents cités:
- EP-A1- 0 739 999
- EP-A1- 1 052 027
- FR-A1- 2 763 259
- US-A- 3 719 739
- US-A- 3 882 050
- US-A- 4 377 454
- US-A- 4 416 742
- US-A- 4 472 533
- US-A- 4 743 577
- US-A- 5 318 688
- US-A1- 2001 042 344
- US-A1- 2004 023 798
- US-B1- 6 676 919

## Description

La présente invention concerne l'utilisation pour l'hydrogénation sélective de coupes d'hydrocarbures contenant des alcènes et une quantité substantielle de diènes et/ou alcynes ayant de 2 à 12 atomes de carbone d'un catalyseur métallique supporté comprenant du platine et/ou du palladium.

De nombreux procédés industriels de transformation de composés organiques incluent une ou plusieurs étapes d'hydrogénation, lesquelles mettent en oeuvre des catalyseurs dits « hydrogénants », qui sont le plus souvent des catalyseurs supportés comprenant un ou plusieurs métaux « hydrogénants » (c'est à dire des métaux du groupe du platine) déposés sur un support inorganique.

De tels catalyseurs sont par exemple employés dans l'industrie pétrolière ou pétrochimique, afin d'hydrogéner les hydrocarbures insaturés, c'est-à-dire des hydrocarbures comprenant des insaturations notamment acétyléniques (triples liaisons), éthyléniques (doubles liaisons), et/ou aromatiques.

Ces catalyseurs doivent être particulièrement actifs, c'est-à-dire promouvoir avec le meilleur rendement possible la ou les réaction(s) recherchée(s), tout en étant sélectifs, c'est-à-dire en évitant les réactions secondaires non désirées.

Un procédé particulièrement délicat à contrôler est l'hydrogénation sélective des oléfines légères.

Les oléfines légères (c'est-à-dire les alcènes comprenant, typiquement, de 2 à 6 atomes de carbone), sont des matières premières importantes employées dans la préparation des nombreux polymères et produits chimiques.

Elles sont le plus souvent issues du raffinage du pétrole, notamment du craquage ou de la pyrolyse de coupes plus lourdes. Cependant, les coupes d'oléfines légères comportent souvent des quantités plus ou moins importantes d'acétylène et/ou de diènes, qui sont des composés indésirables qui compromettent l'utilisation ultérieure des oléfines.

Ces composés sont donc éliminés des coupes d'oléfines légères, par des réactions dites d'hydrogénation sélective. Les catalyseurs employés dans ces réactions doivent être particulièrement sélectifs, en ce qu'ils doivent promouvoir l'hydrogénation de l'acétylène et/ou des diènes, sans hydrogéner les oléfines. Il s'agit en général de catalyseurs dont la phase active est à base de palladium déposé sur un support inorganique de type silice ou alumine. La phase active peut aussi être déposée sur un support métallique (US 4 472 533 A).

Poursuivant ses recherches dans le domaine des catalyseurs hydrogénants, la Demanderesse a maintenant découvert que l'on pouvait préparer des catalyseurs particulièrement actifs, et particulièrement sélectifs en déposant, sur un support métallique, des couches particulières de métaux spécifiques. Selon l'invention, de tels catalyseurs sont préparés par déposition électrolytique.

Ainsi, la présente invention a pour objet l'utilisation pour l'hydrogénation sélective de coupes d'hydrocarbures contenant des alcènes et une quantité substantielle de diènes et/ou alcynes ayant de 2 à 12 atomes de carbone d'un catalyseur métallique supporté obtenu par une procédé comprenant les étapes suivantes:
a) dépôt électrolytique d'une couche de nickel sur un support métallique, puis
e) dépôt électrolytique d'une couche d'un ou plusieurs métaux additionnels choisis parmi l'or, l'argent, le zinc, le manganèse et le cuivre, puis
b) dépôt électrolytique d'une couche supérieure de platine et/ou de palladium.

Le procédé de préparation du catalyseur permet de préparer des catalyseurs hydrogénants particulièrement actifs et sélectifs. Ces catalyseurs sont particulièrement efficaces pour l'hydrogénation sélective d'hydrocarbures insaturés, et notamment pour l'hydrogénation sélective d'oléfines légères. Le caractère spécifique principal est la sélectivité, à savoir la propension à effectuer une hydrogénation complète des dioléfines et des acétylènes (triples liaisons), sans hydrogéner les oléfines.

Ces catalyseurs présentent également l'avantage d'être particulièrement résistants à l'usage et/ou à l'abrasion. Notamment, la couche supérieure de platine et/ou de palladium adhère particulièrement bien au catalyseur, le métal s'élimine moins au cours du temps que dans certains catalyseurs de l'art antérieur, ce qui garantit une bonne longévité du catalyseur, et un excellent maintien de son niveau d'activité au cours du temps.

Ces catalyseurs présentent également l'avantage de pouvoir être préparés sur des supports de faible coût, notamment des supports déjà utilisés dans d'autres applications, comme des anneaux de Raschig, des anneaux de Pall, des éléments de garnissage de colonnes de distillation, des fils métalliques, des toiles tissées à partir de fils métalliques, des grilles métalliques...

Un autre avantage de ces catalyseurs réside dans leur simplicité de recyclage à la fin de leur vie. Du fait de leur composition essentiellement voire totalement métallique le schéma de récupération du métal précieux s'en trouve simplifié, alors que les catalyseurs classiquement employés dans l'art antérieur consistent en une très faible quantité de métal déposé sur un support inorganique (alumine, silice...), ce qui rend plus difficile le recyclage des métaux.

Les catalyseurs sont préparés à partir d'un support métallique, c'est-à-dire constitué d'un matériau (éventuellement composite) contenant un ou plusieurs métaux.

L'on peut employer à cet effet tout support qui contient une quantité de métaux et/ou d'alliages métalliques suffisante pour que le support soit électro-conducteur, de manière à permettre le dépôt électrolytique de couches métalliques à sa surface.

De préférence, le support est constitué d'un ou plusieurs métaux choisis parmi le fer, le chrome, le nickel, le cuivre, le molybdène, l'aluminium, le zinc, et les alliages de ces métaux.

De manière plus préférée, le support contient au moins 50% en poids de fer et/ou d'alliage de fer comme en particulier les aciers. On emploie de manière particulièrement préférée les aciers inoxydables, qui présentent l'avantage d'être largement disponibles commercialement sous différentes formes géométriques.

Néanmoins d'autres types particuliers d'alliages peuvent également être employés. A titre d'exemple, on citera le Monel, alliage de nickel et cuivre, qui convient également pour cette application. Il présente néanmoins l'inconvénient d'être moins disponible commercialement sous les formes géométriques désirées.

Ainsi, selon un mode de réalisation préféré de l'invention, le support est constitué d'acier inoxydable.

Le procédé de préparation comprend au moins trois étapes a), e) et b), qui consistent chacune à effectuer un dépôt électrolytique d'une couche métallique particulière sur le support métallique.

De manière connue en soi, on désigne par dépôt électrolytique, le fait d'effectuer, au moyen d'un courant électrique, le dépôt d'un ou plusieurs métaux à la surface du support métallique, à partir d'une solution de cations du ou des métaux à déposer. Cette technique, également connue de l'homme du métier sous les noms de galvanoplastie ou électrodéposition, utilise le principe de l'électrolyse.

Pour cela, le support métallique est avantageusement immergé dans une solution des cations du ou des métaux à déposer, et constitue la cathode du système électrolytique. Une ou plusieurs anode(s) par exemple à base de carbone est (sont) également immergée(s) dans la solution, et l'on fait circuler un courant électrique entre la ou les anode(s) et la cathode, jusqu'à l'obtention sur la cathode (c'est-à-dire, sur le support) d'une couche métallique ayant l'épaisseur souhaitée.

Le procédé de préparation comprend ainsi une étape a) de dépôt électrolytique d'une couche de nickel sur le support métallique.

Un tel dépôt est avantageusement effectué à partir d'une solution aqueuse d'un ou plusieurs sels de nickel, qui peuvent être tout particulièrement choisis parmi le chlorure de nickel (NiCl₂), le sulfate de nickel (NiSO₄) et le sulfamate de nickel Ni(NH₂SO₃)₂, et de préférence à partir d'une solution aqueuse de chlorure de nickel (NiCl₂). De manière particulièrement préférée, on utilise une solution aqueuse de chlorure de nickel (NiCl₂), ayant un pH acide et contenant de l'acide chlorhydrique (HCl).

La solution peut contenir d'autres éléments, comme par exemple, l'acide borique, des acides organiques, des sucres, des tensioactifs, des sels minéraux (chlorures, cyanures...).

L'étape a) est typiquement effectuée à température modérée, de 10 à 100°C, de préférence de 20 à 90°C et à pression atmosphérique (760 mm Hg).

Selon l'invention, la couche de nickel est avantageusement déposée directement sur ledit support métallique, et le recouvre entièrement.

Son épaisseur est de préférence de 0,01 µm à 10 µm, et plus préférentiellement de 0,05 µm à 2 µm.

Le procédé de préparation comprend également une étape b) de dépôt électrolytique d'une couche supérieure de platine et/ou de palladium.

Selon un mode de réalisation particulièrement préféré, l'étape b) consiste à déposer une couche comprenant du palladium, et encore plus préférentiellement l'étape b) consiste à déposer une couche constituée de palladium.

Le dépôt électrolytique de l'étape b) est avantageusement effectué à partir d'une solution aqueuse contenant du platine et/ou du palladium, de préférence sous forme de sel(s) et/ou de complexe(s).

De tels composés peuvent être notamment choisis parmi :
- Les sels et complexes, notamment halogénés, aminés ou amino-nitrés, du platine, et en particulier : Pt(NH₃)₂(NO₂)₂, Pt(NH₃)₄(NO₂)₂, Pt(NH₃)₄Cl₂ ;
- Les sels et complexes, notamment halogénés, aminés ou amino-nitrés, du palladium, et de préférence les sels de palladium ethylènediamine et de palladium tétramine, comme Pd(NH₃)₂(NO₂)₂, Pd(NH₃)₄(NO₂)₂, Pd(NH₃)₄Cl₂.

Ces composés sont mis en solution et appliqués à un pH de préférence supérieur à 5.

On emploie plus préférentiellement une solution aqueuse d'un ou plusieurs sels et/ou complexes du palladium, et de manière particulièrement préférée une solution aqueuse de sel de palladium tétramine.

L'étape b) est typiquement effectuée à température modérée, entre 10 et 100°C, de préférence entre 30 et 90°C et à pression atmosphérique.

Selon l'invention, la couche de platine et/ou de palladium est la couche supérieure du catalyseur, c'est-à-dire qu'elle constitue la surface extérieure du catalyseur. Cela signifie en particulier qu'aucun autre composé, notamment aucun autre type de métal n'est déposé sur cette couche.

Le dépôt est avantageusement effectué à l'étape b) de manière à recouvrir toute la surface du catalyseur, c'est-à-dire que la couche de platine et/ou de palladium s'étend sur toute la surface extérieure du catalyseur, et recouvre ainsi intégralement la ou les couches inférieures.

L'épaisseur de cette couche est de préférence de 0,01 à 1 µm et de préférence de 0,02 à 0,1 µm.

Avant l'étape a) de dépôt de la première couche métallique, il peut être nécessaire d'effectuer un ou plusieurs traitements préalables de préparation de la surface du support, selon des techniques connues de l'art antérieur. En particulier, les éventuelles traces de corps organique doivent de préférence être éliminées. A cet effet un nettoyage du support au solvant organique, comme l'acétone, ou un nettoyage par des produits alcalins, comme la soude, peuvent être effectués. Des techniques comme le nettoyage en bain ultrasonique peuvent également être employées. Ensuite un décapage chimique, par exemple à l'acide chlorhydrique, peut être avantageux pour favoriser l'adhésion sur le support des couches de dépôt ultérieur

A l'issue des étapes a) et b), le catalyseur est de préférence lavé, par exemple à l'eau à température ambiante. Il peut être également séché pour éliminer l'eau à des températures de 20 à 100°C.

On réalise entre l'étape a) et l'étape b), une ou plusieurs étapes intermédiaires, et notamment une ou plusieurs étapes de dépôt d'une ou plusieurs couches métalliques intermédiaires. De tels dépôts peuvent être avantageusement réalisés également par dépôt électrolytique.

Le procédé de préparation comprend, entre l'étape a) et l'étape b), une étape intermédiaire e) de dépôt électrolytique d'une couche d'un ou plusieurs métaux additionnels, choisis parmi les métaux qui sont catalytiquement neutres par rapport aux réactions d'hydrogénation d'hydrocarbures, et en particulier par rapport aux réactions d'hydrogénation des alcènes (comportant une ou plusieurs doubles liaisons) et des alcynes. Ces métaux additionnels sont choisis parmi l'or, l'argent, le zinc, le manganèse et le cuivre.

Le dépôt d'une telle couche intermédiaire permet de recouvrir d'un métal catalytiquement neutre la couche inférieure de nickel, qui peut avoir une influence négative sur le rendement et surtout la sélectivité des réactions d'hydrogénation d'hydrocarbures.

Il est ainsi possible de réduire au maximum la quantité de platine et/ou de palladium constituant la couche supérieure, qui sont des métaux particulièrement coûteux, tout en conservant de bonnes performances en termes d'activité et de sélectivité du catalyseur, quand bien même la couche supérieure viendrait à présenter localement (c'est-à-dire, en certains points du catalyseur) une épaisseur insuffisante par exemple en raison de l'usure du catalyseur.

Selon un mode de réalisation particulièrement préféré, ce ou ces métaux additionnels sont choisis parmi les métaux colorés, c'est-à-dire ceux qui présentent une couleur différente de la couleur du nickel (blanc argenté).

Ainsi, on préfère employer l'or et le cuivre, et tout particulièrement le cuivre.

Un tel dépôt intermédiaire d'une couche métallique colorée présente l'avantage de permettre de contrôler de manière particulièrement simple, notamment visuellement, l'étape b) de dépôt de la couche supérieure de platine et/ou de palladium.

En effet, il importe de s'assurer à l'étape b) que la couche supérieure de platine et/ou de palladium recouvre de manière complète toute la surface du catalyseur, afin de garantir une efficacité optimale du catalyseur, tout en employant la quantité de métal strictement nécessaire pour atteindre cet objectif, d'autant plus que le platine et le palladium sont des métaux particulièrement coûteux.

La présence d'une couche métallique intermédiaire colorée permet entre autres de contrôler visuellement l'avancement du processus de dépôt électrolytique au cours de l'étape b), et ainsi de stopper celui-ci dès que la couche métallique colorée n'est plus visible, ce qui indique que la surface du catalyseur est entièrement revêtue de platine et/ou de palladium. Ce contrôle visuel est facile à effectuer, et permet de s'affranchir de méthodes plus complexes de mesure d'épaisseur.

Il est toutefois parfaitement possible de contrôler l'avancement de l'étape b) par d'autres méthodes, telles qu'en particulier la microscopie électronique. Il est également possible de mettre en oeuvre de méthodes telles que la spectroscopie de photoélectrons X (ou XPS, de l'anglais « X-Ray Photoelectron Spectrosopy »)

L'étape intermédiaire e) est avantageusement effectuée à partir d'une solution aqueuse d'un ou plusieurs sels dudit ou desdits métaux additionnels. Dans le cas du cuivre, ce dépôt est de préférence effectué à partir d'une solution aqueuse de sels de cuivre, qui peuvent être tout particulièrement choisis parmi le chlorure de cuivre (CuCl₂), l'acétate de cuivre (CH₃COO)₂Cu, le fluoborate de cuivre Cu(BF₄)₂, le sulfate de cuivre (CuSO₄), et leurs mélanges.

De manière particulièrement préférée, on utilise une solution diluée de sulfate de cuivre (CuSO₄) contenant également de l'acide sulfurique.

La solution peut contenir d'autres éléments, comme par exemple, l'acide borique, des acides organiques, des sucres, des tensio-actifs, des sels minéraux (chlorures, cyanures...).

L'étape e) est typiquement effectuée à température modérée, entre 10 et 100°C, de préférence entre 30 et 90°C.

La quantité de métal additionnel déposé au cours de l'étape e) correspond avantageusement à la quantité minimale requise pour obtenir un recouvrement complet de la couche de nickel déposée à l'étape a). L'obtention d'un tel recouvrement complet de la couche de nickel peut également être contrôlée visuellement dans le cas d'un métal additionnel coloré (tel que l'or ou le cuivre), ou par les méthodes telles que la microscopie électronique, la spectroscopie de photoélectrons X.

L'épaisseur de la couche de métal additionnel déposé au cours de l'étape e) va de préférence de 0,01 µm à 10 µm, et plus préférentiellement de 0,05 µm à 2 µm.

Le catalyseur métallique supporté, susceptible d'être obtenu par le procédé tel que décrit ci-avant se présente avantageusement sous forme de particules solides de petite taille telles que des billes, des particules plus ou moins cylindriques, ou encore sous forme de fils, de toiles, de grilles ou toute autre forme appropriée.

La forme du catalyseur dépend en général de celle du support métallique de départ, et il convient donc d'utiliser un support ayant la forme souhaitée pour le catalyseur final.

Le catalyseur peut être notamment utilisé dans des réactions d'hydrogénation d'hydrocarbures insaturés.

Selon l'invention il est employé dans les procédés d'hydrogénation sélective d'oléfines légères, c'est-à-dire pour l'hydrogénation sélective des coupes d'hydrocarbures contenant des alcènes et une quantité substantielle de diènes et/ou d'alcynes ayant de 2 à 12 atomes de carbone.

Ces procédés peuvent traiter des charges de différents types, comme en particulier l'essence de vapocraquage (ou « steam cracking ») encore appelée pygas (« pyrolisis gasoline »), ainsi que la coupe en C4, la coupe en C3 ou la coupe en C2 issue d'un tel procédé de vapocraquage.

Dans un exemple particulier, le catalyseur selon l'invention est utilisé pour l'hydrogénation sélective d'une coupe d'hydrocarbures contenant de l'acétylène, telle que la coupe en C2 issue du vaporéformage, avec pour but de convertir l'acétylène en éthylène en présence d'hydrogène. Le catalyseur selon l'invention permet ainsi de convertir l'acétylène en éthylène sans hydrogéner ce dernier composé, ni les autres oléfines éventuellement présentes dans la charge.

Dans ces procédés d'hydrogénation sélective, la charge liquide et/ou gazeuse est mise en contact avec de l'hydrogène et le catalyseur selon l'invention à une température allant de 30 à 200°C et à une pression allant de 10 à 40 bars (10⁵ à 4.10⁶ Pa - 0,1 à 4 MPa).

Dans le cas particulier d'hydrogénation de l'acétylène en éthylène, les conditions sont de préférence une température allant de 30 à 100°C et une pression allant de 10 à 40 bars (10⁵ à 4.10⁶ Pa - 0,1 à 4 MPa).

Au cours de son utilisation, le catalyseur selon l'invention est susceptible de se désactiver progressivement. Il peut cependant être régénéré ou réactivé, de manière facile et efficace. L'on peut ainsi effectuer un ou plusieurs traitements du catalyseur sous atmosphère réductrice (hydrogène) ou sous atmosphère oxydante (air) à une température supérieure à 100°C et de préférence comprise entre 200 et 500°C peuvent permettre, ce qui permet de redonner une activité accrue à un catalyseur selon l'invention désactivé.

Les exemples qui suivent sont donnés à titre purement illustratif de la présente invention.

### EXEMPLES :

### Préparation des supports catalytiques :

Les catalyseurs décrits dans les exemples ci-après ont été préparés sur un support constitué d'éléments de garnissage métallique pour colonne de distillation commercialisés sous l'appellation « Pro-Pak Protruded Metal Distillation Packing » par la société Cannon Instrument Company. La taille de ces éléments est de 0,16 inch (4,064 mm) et ils sont constitués d'acier inox 316.

La préparation du support est effectuée par traitement de leur surface, en deux étapes : lavage au solvant puis traitement acide.

La première étape est un dégraissage au solvant. Celle-ci permet d'enlever des dépôts organiques en surface. Une quantité de 1 g de support est immergée dans un bécher rempli de 20 ml d'acétone pendant 2 minutes sous agitation, puis sortie et séchée dans un four à moufle à 100°C pendant 5 minutes.

La seconde étape est un décapage acide. Elle permet d'améliorer l'adhérence des couches métalliques déposées par d'électrodéposition.

Le support dégraissé est placé dans un bécher de 20 ml d'acide chlorhydrique à 15% (dilué à l'eau distillée) préalablement chauffé à 60°C, et laissé 3 minutes.

Le support est ensuite immédiatement transféré dans le bain suivant d'électrodéposition, en évitant toute exposition prolongée à l'air.

### Exemple 1: Comparatif : dépôt direct de palladium sur acier - mauvaise adhérence - pas de test d'activité

Un montage conventionnel d'électrodéposition a été utilisé avec un système à courant continu. Un catalyseur a été préparé de la manière suivante :
Une quantité de 1g de support préparé de la manière décrite ci-avant est immergée dans 200ml d'une solution de chlorure de palladium tétramine à 2,5 g de Pd par litre, le support constituant la cathode du système. L'anode est une plaque de titane recouverte de platine. La solution est perpétuellement agitée pendant l'opération. Une tension de 1,6 Volt est appliquée pendant 5 minutes à une température de 50°C.

Le catalyseur ainsi obtenu est rincé par 200ml d'eau distillée.

L'application dans ces conditions a montré visuellement que la couche de palladium n'est pas adhérente : le simple fait de la frotter avec un chiffon l'enlève partiellement, ce qui fait conclure que ces conditions ne sont pas satisfaisantes.

### Exemple 2: Comparatif : catalyseur au nickel sur acier - mauvaise activité

Le même montage d'électrodéposition et la même matière première qu'à l'exemple 1 ont été utilisés. Un catalyseur a été préparé de la manière suivante :
Une quantité de 1g de support est immergée dans 200ml d'une solution acide de chlorure de nickel, contenant 150 g/L d'HCl et 50g/L de Nickel (exprimé en Ni), à la température de 20°C. Le support constitue une électrode, l'autre étant une barre de Nickel.

L'électrodéposition s'effectue en 3 temps distincts : d'abord l'application d'un voltage de 1 Volt en branchant le support en anode pendant 2 minutes, ensuite en inversant les électrodes pendant 3 minutes à 1,9 V, puis en inversant à nouveau pendant 3 minutes à 1,9V.

Le catalyseur ainsi obtenu est rincé par 200ml d'eau distillée.

Le catalyseur ainsi obtenu a été testé sur la réaction sélective d'hydrogénation de l'isoprène.

### Exemple 3: Comparatif: catalyseur au palladium sur nickel sur acier, teneur moyenne en palladium

Le support recouvert de nickel obtenu dans l'exemple 2 a été soumis à un dépôt de palladium dans les conditions telles que décrites à l'exemple 1 :
Une quantité de 1g du support recouvert de nickel obtenu dans l'exemple 2 est immergée dans 200ml d'une solution de chlorure de palladium tétramine à 2,5 g de Pd par litre, et constitue la cathode du système. L'anode est une plaque de titane recouverte de platine. Une tension de 1,6 Volt est appliquée pendant 2 minutes à une température de 50°C.

Le catalyseur ainsi obtenu est rincé par 200ml d'eau distillée.

Le catalyseur ainsi obtenu a été testé sur la réaction d'hydrogénation sélective de l'isoprène.

### Exemple 4: Comparatif: catalyseur au palladium sur nickel sur acier, haute teneur en palladium

Le catalyseur de cet exemple a été préparé dans les mêmes conditions que l'exemple 3 ci-avant, avec la différence que le temps de dépôt a été doublé : il est passé de 2 minutes à 4 minutes, afin d'augmenter la quantité de palladium déposé.

Le catalyseur ainsi obtenu a été testé sur la réaction sélective d'hydrogénation de l'isoprène.

### Exemple 5 selon l'invention : catalyseur au palladium sur cuivre sur nickel sur acier, teneur faible en palladium

Le support recouvert de nickel obtenu dans l'exemple 2 a été soumis à un dépôt de cuivre préalable au dépôt de palladium (ce dernier étant réalisé dans les mêmes conditions qu'à l'exemple 1) :
Une quantité de 1g du support recouvert de nickel obtenu dans l'exemple 2 est immergée dans 200ml d'une solution acide de sulfate de cuivre, contenant 50 g/L d'acide sulfurique (H₂SO₄) et 50g/L de cuivre (exprimé en Cu), à la température de 20°C. Ce support constitue une électrode, l'autre étant une barre de cuivre. L'électrodéposition s'effectue en 3 temps distincts : d'abord l'application d'un voltage de 1,5 Volt en branchant le support en anode pendant 1 minute, ensuite en inversant les électrodes pendant 3 minutes à 1,5 V, puis en inversant à nouveau pendant 2 minutes à 1,5 V.

Le support recouvert de cuivre ainsi obtenu, de couleur rouge, est rincé par 200ml d'eau distillée.

Une quantité de 1g de ce support recouvert de cuivre est ensuite immergée dans 200ml d'une solution de chlorure de palladium tétramine à 2,5 g de Pd par litre, et constitue la cathode du système. L'anode est une plaque de titane recouverte de platine. Une tension de 1,6 Volt est appliquée pendant 2 minutes à une température de 50°C.

Le catalyseur ainsi obtenu est rincé par 200ml d'eau distillée.

Le catalyseur ainsi obtenu a été testé sur la réaction d'hydrogénation sélective de l'isoprène.

### Test d'activité par hydrogénation sélective de l'isoprène :

### - Description du test d'activité employé :

Les différents catalyseurs préparés ci-après ont été soumis à un test catalytique d'hydrogénation sélective d'une molécule modèle qui est l'isoprène, dans un autoclave sous pression d'hydrogène. L'isoprène est une dioléfine qui peut être hydrogénée une première fois en isopentène (plus précisément 3 isomères, le 2 méthyl 1 butène, le 2 méthyl 2 butène, le 3 méthyl 1 butène), puis une deuxième fois en isopentane.

Le poids de catalyseur dans l'autoclave est de 0,1g.

La charge employée est constituée d'isoprène à 5% en poids, dilué dans l'heptane.

La température est de 100°C, la pression d'hydrogène maintenue à 0,7 MPa.

Le test consiste à déterminer les trois valeurs suivantes:
- T1 est le temps en minutes nécessaire pour que la concentration en isoprène descende à 1% en poids dans le milieu réactionnel ;
- T2 est le temps en minutes nécessaire pour que la concentration en isopentane atteigne 2% en poids dans le milieu réactionnel ;
- la sélectivité est le ratio molaire (en %) isopentène/(isopentène + isopentane) au point T1, déterminée au moment où la concentration d'isoprène a atteint 1.0% en poids.

Le test a été calibré avec un catalyseur commercial de type E series de la société Chevron Phillips (référence).

Le premier critère d'évaluation de l'activité des catalyseurs pour l'hydrogénation sélective est la fenêtre opératoire, ou Delta T, qui correspond à T2 - T1. Plus la valeur Delta T est importante, meilleur est le catalyseur pour l'hydrogénation sélective de l'isoprène en isopentènes.

Le second critère essentiel est la sélectivité.

Il importe que le catalyseur puisse hydrogéner le plus complètement possible l'isoprène avant de commencer à transformer les isopentènes en isopentane (cette dernière réaction correspondant à une baisse de rendement en produit recherché).

### - Résultats obtenus :

Les résultats des tests sont résumés dans le tableau ci-dessous :

| **Exemple** | | **Catalyseur Technique de dépôt** | **T1 (min)** | **T2 (min)** | **Delta T (min)** | **Sélectivité (%)** |
|---|---|---|---|---|---|---|
| - | comparatif | Catalyseur commercial de référence | 92 | 130 | 38 | 96 |
| 2 | comparatif | Ni sur acier | >240 | >240 | - | - |
| 3 | comparatif | Pd sur Ni sur acier - moyenne teneur en Pd | 93 | 180 | 87 | 78 |
| 4 | comparatif | Pd sur Ni sur acier - haute teneur en Pd | 94 | 204 | 114 | 99 |
| 5 | invention | Pd sur Cu sur Ni sur acier - basse teneur en Pd | 73 | 210 | 137 | 100 |

Le critère principal de qualité de ces catalyseurs pour l'application d'hydrogénation sélective n'est pas tant l'activité, mais plutôt la facilité d'opération, représentée par la fenêtre opératoire, ainsi que la sélectivité.

Dans ce test, qui est simplifié par rapport à la réalité de vraies conditions opératoires, on simule l'activité par le temps T1. Plus le temps T1 est court, plus le catalyseur est actif.

On simule la fenêtre opératoire (c'est-à-dire, l'écart de température observé entre le début d'hydrogénation de l'impureté alcyne (acétylène) et le début d'hydrogénation du composant majoritaire alcène (éthylène)), par l'écart de temps entre T1 et T2, obtenu sur la dioléfine modèle, l'isoprène. Plus le Delta T (T2 - T1) est grand, plus le catalyseur aura une plage opératoire importante, et donc sera facile à opérer de façon sélective pour la production d'oléfines.

Enfin la sélectivité permet d'évaluer directement la propension à ne pas effectuer d'hydrogénation complète en alcane pour un niveau de conversion élevé d'isoprène.

Les résultats figurant dans le tableau ci-avant montrent que le catalyseur de l'exemple 2 (support recouvert de nickel) est insuffisamment actif.

Le catalyseur de l'exemple 5 selon l'invention présente des performances améliorées par rapport à l'art antérieur (catalyseur commercial de référence) et aux catalyseurs des exemples comparatifs 3 et 4.

## Revendications

1. Utilisation pour l'hydrogénation sélective de coupes d'hydrocarbures contenant des alcènes et une quantité substantielle de diènes et/ou d'alcynes ayant de 2 à 12 atomes de carbone d'un catalyseur métallique supporté obtenu par un procédé comprenant les étapes suivantes :
a) dépôt électrolytique d'une couche de nickel sur un support métallique, puis
e) dépôt électrolytique d'une couche d'un ou plusieurs métaux additionnels choisis parmi l'or, l'argent, le zinc, le manganèse et le cuivre, puis
b) dépôt électrolytique d'une couche supérieure de platine et/ou de palladium.

2. Utilisation selon la revendication 1, **caractérisée en ce que** le support est constitué d'un ou plusieurs métaux choisis parmi le fer, le chrome, le nickel, le cuivre, le molybdène, l'aluminium, le zinc, et les alliages de ces métaux.

3. Utilisation selon la revendication précédente, **caractérisée en ce que** le support contient au moins 50% en poids de fer et/ou d'alliage de fer, et de préférence le support est constitué d'acier inoxydable.

4. Utilisation selon l'une quelconque des revendications précédentes, **caractérisée en ce que** le dépôt électrolytique de l'étape a) est effectué à partir d'une solution aqueuse d'un ou plusieurs sels de nickel choisis parmi le chlorure de nickel (NiCl₂), le sulfate de nickel (NiSO₄) et le sulfamate de nickel Ni(NH₂SO₃)₂, et de préférence à partir d'une solution aqueuse de chlorure de nickel (NiCl₂).

5. Utilisation selon l'une quelconque des revendications précédentes, **caractérisée en ce que** l'épaisseur de la couche de nickel va de 0,01 µm à 10 µm, et de préférence de 0,05 µm à 2 µm.

6. Utilisation selon l'une quelconque des revendications précédentes, **caractérisée en ce que** le dépôt électrolytique de l'étape b) est effectué à partir d'une solution aqueuse contenant un ou plusieurs sels et/ou complexes du platine et/ou du palladium, choisis parmi :
- les sels et complexes, notamment halogénés, aminés ou amino-nitrés, du platine, et en particulier : Pt(NH₃)₂(NO₂)₂, Pt(NH₃)₄(NO₂)₂, Pt(NH₃)₄Cl₂ ;
- Les sels et complexes, notamment halogénés, aminés ou amino-nitrés, du palladium, et de préférence les sels de palladium ethylènediamine et de palladium tétramine, comme Pd(NH₃)₂(NO₂)₂, Pd(NH₃)₄(NO₂)₂, Pd(NH₃)₄Cl₂.
et de préférence à partir d'une solution aqueuse d'un ou plusieurs sels et/ou complexes du palladium, et de manière particulièrement préférée une solution aqueuse de sel de palladium tétramine.

7. Utilisation selon l'une quelconque des revendications précédentes, **caractérisée en ce que** l'étape b) consiste à déposer une couche constituée de palladium.

8. Utilisation selon l'une quelconque des revendications précédentes, **caractérisée en ce que** l'épaisseur de la couche de platine et/ou de palladium va de 0,01 à 1 µm et de préférence de 0,02, à 0,1 µm.

9. Utilisation selon l'une quelconque des revendications précédentes, **caractérisée en ce que** ledit ou lesdits métaux additionnels sont choisis parmi les métaux colorés, de préférence parmi l'or et le cuivre, et plus préférentiellement le cuivre.

10. Utilisation selon l'une quelconque des revendications précédentes, pour l'hydrogénation sélective d'oléfines légères, de préférence pour l'hydrogénation sélective d'acétylène en éthylène.

## Patentansprüche

1. Verwendung eines geträgerten Metallkatalysators, der durch ein Verfahren, das folgende Schritte umfasst, erhalten wird:
a) elektrolytische Abscheidung einer Nickelschicht auf einem Metallträger, dann
e) elektrolytische Abscheidung einer Schicht aus einem oder mehreren zusätzlichen Metallen, die aus Gold, Silber, Zink, Mangan und Kupfer ausgewählt sind, dann
b) elektrolytische Abscheidung einer oberen Schicht aus Platin und/oder Palladium,
zur selektiven Hydrierung von Kohlenwasserstoffschnitten, die Alkene und eine wesentliche Menge von Dienen und/oder Alkinen mit 2 bis 12 Kohlenstoffatomen enthalten.

2. Verwendung nach Anspruch 1, **dadurch gekennzeichnet, dass** der Träger aus einem oder mehreren Metallen besteht, die aus Eisen, Chrom, Nickel, Kupfer, Molybdän, Aluminium, Zink und Legierungen dieser Metalle ausgewählt sind.

3. Verwendung nach einem der vorhergehenden Ansprüche, **dadurch gekennzeichnet, dass** der Träger mindestens 50 Gew.-% Eisen und/oder Eisenlegierung enthält und vorzugsweise aus nichtrostendem Stahl besteht.

4. Verwendung nach einem der vorhergehenden Ansprüche, **dadurch gekennzeichnet, dass** die elektrolytische Abscheidung von Schritt a) ausgehend von einer wässrigen Lösung eines oder mehrerer Nickelsalze, die aus Nickelchlorid (NiCl₂), Nickelsulfat (NiSO₄) und Nickelsulfamat Ni(NH₂SO₃)₂ ausgewählt sind, und vorzugsweise ausgehend von einer wässrigen Lösung von Nickelchlorid (NiCl₂) durchgeführt wird.

5. Verwendung nach einem der vorhergehenden Ansprüche, **dadurch gekennzeichnet, dass** die Dicke der Nickelschicht im Bereich von 0,01 µm bis 10 µm und vorzugsweise von 0,05 µm bis 2 µm liegt.

6. Verwendung nach einem der vorhergehenden Ansprüche, **dadurch gekennzeichnet, dass** die elektrolytische Abscheidung von Schritt b) ausgehend von einer wässrigen Lösung, die ein oder mehrere Salze und/oder Komplexe von Platin und/oder Palladium, die aus
- Salzen und Komplexen, insbesondere Halogen-, Amino- oder Aminonitro-Salzen und -Komplexen von Platin und insbesondere Pt(NH₃)₂(NO₂)₂, Pt(NH₃)₄(NO₂)₂ und Pt(NH₃)₄Cl₂,
- Salzen und Komplexen, insbesondere Halogen-, Amino- oder Aminonitro-Salzen und -Komplexen von Palladium und bevorzugt Palladium-Ethylendiamin- und Palladium-Tetramin-Salze und insbesondere Pd(NH₃)₂(NO₂)₂, Pd(NH₃)₄(NO₂)₂ und Pd(NH₃)₄Cl₂, ausgewählt sind, enthält,
und vorzugsweise ausgehend von einer wässrigen Lösung eines oder mehrerer Salze und/oder Komplexe von Palladium und besonders bevorzugt einer wässrigen Lösung von Palladium-Tetramin-Salz durchgeführt wird.

7. Verwendung nach einem der vorhergehenden Ansprüche, **dadurch gekennzeichnet, dass** der Schritt b) aus der Abscheidung einer Schicht aus Palladium besteht.

8. Verwendung nach einem der vorhergehenden Ansprüche, **dadurch gekennzeichnet, dass** die Dicke der Platin- und/oder Palladiumschicht im Bereich von 0,01 bis 1 µm und vorzugsweise von 0,02 bis 0,1 µm liegt.

9. Verwendung nach einem der vorhergehenden Ansprüche, **dadurch gekennzeichnet, dass** das zusätzliche Metall bzw. die zusätzlichen Metalle aus farbigen Metallen, vorzugsweise aus Gold und Kupfer und weiter bevorzugt Kupfer ausgewählt ist bzw. sind.

10. Verwendung nach einem der vorhergehenden Ansprüche zur selektiven Hydrierung von leichten Olefinen, vorzugsweise zur selektiven Hydrierung von Acetylen zu Ethylen.

## Claims

1. Use, for the selective hydrogenation of hydrocarbon cuts containing alkenes and a substantial amount of dienes and/or of alkynes having from 2 to 12 carbon atoms, of a supported metal catalyst obtained by a process comprising the following stages:
a) electrolytic deposition of a nickel layer on a metal support, then
e) electrolytic deposition of a layer of one or more additional metals chosen from gold, silver, zinc, manganese and copper, then
b) electrolytic deposition of an upper layer of platinum and/or of palladium.

2. Use according to Claim 1, **characterized in that** the support consists of one or more metals chosen from iron, chromium, nickel, copper, molybdenum, aluminium, zinc and the alloys of these metals.

3. Use according to the preceding claim, **characterized in that** the support contains at least 50% by weight of iron and/or of iron alloy, and preferably the support consists of stainless steel.

4. Use according to any one of the preceding claims, **characterized in that** the electrolytic deposition of stage a) is carried out starting from an aqueous solution of one or more nickel salts chosen from nickel chloride (NiCl₂), nickel sulphate (NiSO₄) and nickel sulfamate Ni(NH₂SO₃)₂, and preferably starting from an aqueous solution of nickel chloride (NiCl₂).

5. Use according to any one of the preceding claims, **characterized in that** the thickness of the nickel layer ranges from 0.01 µm to 10 µm and preferably from 0.05 µm to 2 µm.

6. Use according to any one of the preceding claims, **characterized in that** the electrolytic deposition of stage b) is carried out starting from an aqueous solution containing one or more salts and/or complexes of platinum and/or of palladium, chosen from:
- salts and complexes, in particular halogenated, aminated or amino-nitrated, of platinum, and in particular: Pt(NH₃)₂(NO₂)₂, Pt(NH₃)₄(NO₂)₂ or Pt(NH₃)₄Cl₂;
- salts and complexes, in particular halogenated, aminated or amino-nitrated, of palladium, and preferably (ethylenediamine)palladium and tetraaminepalladium salts, such as Pd(NH₃)₂(NO₂)₂, Pd(NH₃)₄(NO₂)₂ or Pd(NH₃)₄Cl₂,
and preferably starting from an aqueous solution of one or more salts and/or complexes of palladium, and particularly preferably an aqueous solution of tetraaminepalladium salt.

7. Use according to any one of the preceding claims, **characterized in that** stage b) consists in depositing a layer consisting of palladium.

8. Use according to any one of the preceding claims, **characterized in that** the thickness of the platinum and/or palladium layer ranges from 0.01 to 1 µm and preferably from 0.02 to 0.1 µm.

9. Use according to any one of the preceding claims, **characterized in that** the said additional metal or metals are chosen from coloured metals, preferably from gold and copper, and more preferably copper.

10. Use according to any one of the preceding claims, for the selective hydrogenation of light olefins, preferably for the selective hydrogenation of acetylene into ethylene.
